# EUROPEAN PATENT SPECIFICATION

(11) **EP 2 730 302 B1**
(45) Date of publication and mention of the grant of the patent: **30.11.2016**
(21) Application number: 11869129.4
(22) Date of filing: 06.07.2011
(51) Int. Cl.: A61M 1/14, A61B 5/0205, A61B 5/022, A61B 5/021, A61B 5/00, G06F 19/00

(54) **HYPOTENSION-PREDICTING DEVICE**
VORRICHTUNG ZUR HYPOTONIE-PROGNOSE
DISPOSITIF DE PRÉDICTION D'HYPOTENSION

(43) Date of publication of application: 14.05.2014
(73) Proprietor: Pioneer Corporation, Kawasaki-shi, Kanagawa 212-0031 (JP)
(72) Inventor: MORI, Shuntaro, Kawasaki-shi Kanagawa 212-0031 (JP); KIMURA, Yoshinori, Kawasaki-shi Kanagawa 212-0031 (JP); ITO, Atsuya, Kawasaki-shi Kanagawa 212-0031 (JP); GOMA, Masaki, Kawasaki-shi Kanagawa 212-0031 (JP)
(74) Representative: Viering, Jentschura & Partner mbB Patent- und Rechtsanwälte
(86) International application number: PCT/JP2011/065503
(87) International publication number: WO 2013/005320

(56) References cited:
- EP-A1- 0 956 815
- EP-A1- 2 679 256
- WO-A2-2007/141246
- JP-A- 11 221 275
- JP-A- 2000 157 499
- JP-A- 2002 369 882
- JP-A- 2003 010 319
- JP-A- 2004 357 784
- US-A- 4 718 891
- US-A1- 2004 254 473
- US-A1- 2009 082 684

## Description

### Technical Field

The present invention relates to a blood pressure decrease prediction apparatus which predicts a decrease in blood pressure of a living body or an organism.

### Background Art

There is a possibility of a decrease in a patient's blood pressure during artificial dialysis, for example, because of a decrease in circulating blood volume due to removal of water. If the blood pressure decreases, the patient may be in a state of shock, and the decrease in blood pressure during the artificial dialysis is thus not preferable. So, patent documents 1 and 2 propose a technology which is to promptly judge the decrease in blood pressure of the patient during the artificial dialysis. The patent document 1 discloses a technology which allows a change in blood pressure to be predicted by weighting blood-pressure-change related information to control a rate of water removal during the artificial dialysis on the basis of a result of the predication. Moreover, the patent document 2 discloses a technology which allows an abnormality of the blood pressure to be easily judged in a period of time when the blood pressure easily decreases, by setting, in the period of time when there is a high risk of the decrease in blood pressure of the patient during the dialysis, an abnormality judgment value for judging that the decrease in blood pressure is abnormal to be smaller than the abnormality judgment value in the other period of time.

### Prior Art Document

### Patent Document

Patent document 1: Japanese Patent Application Laid Open No. 2003-10319
Patent document 2: Japanese Patent Application Laid Open No. 2002-369882

Furthermore, document US 2004/0254473 discloses an apparatus for predicting blood pressure by analysing different parameters obtained from a blood flow measurement.

### Disclosure of Invention

### Subject to be Solved by the Invention

However, the technology disclosed in the above described patent document 1 has such a technical problem that judgment accuracy is low because the decrease in blood pressure is judged by using a single evaluation formula from the start to the end of the dialysis. Moreover, since a total value of variations of a plurality of parameter is observed, an acute change in a particular parameter only limitedly influences a judgment result.

Moreover, the technology disclosed in the above described patent document 2 has such a technical problem that it is necessary to obtain individual abnormality judgment values in advance in previous measurement, because each subject or person to be measured has a different abnormality judgment value. Furthermore, the possibility of the decrease in blood pressure may change, even in the same patient, depending on his or her body condition. Thus, at the time of ill health not previously experienced, the abnormality judgment value for predicting the decrease in blood pressure has low reliability, resulting in inaccurate judgment of the decrease in blood pressure, which is technically problematic.

In view of the aforementioned problems, it is therefore an object of the present invention to provide, for example, a blood pressure decrease prediction apparatus which is configured to predict the decrease in blood pressure of the living body, at an early stage and with high accuracy, with causing little burden on the living body.

### Means for Solving the Subject

The above object of the present invention can be achieved by a blood pressure decrease prediction apparatus according to independent claim 1.

These operation and other advantages of the present invention will become more apparent from an embodiment explained below.

### Brief Description of Drawings

[FIG. 1] FIG. 1 is a block diagram illustrating a configuration of a blood pressure decrease prediction apparatus in an example.
[FIG. 2] FIG. 2 is a graph for explaining one example of a method of calculating a pulse rate, a blood flow volume and pulse wave amplitude, based on a blood flow waveform.
[FIG. 3] FIG. 3 is a flowchart illustrating a flow of blood pressure decrease prediction processing.
[FIG. 4] FIG. 4 is a flowchart illustrating a flow of processing in a variation detection sub-routine for the pulse rate.
[FIG. 5] FIG. 5 is a flowchart illustrating a flow of processing in a variation detection sub-routine for the blood flow volume.
[FIG. 6] FIG. 6 is a flowchart illustrating a flow of processing in a variation detection sub-routine for the pulse wave amplitude.
[FIG. 7] FIG. 7 is a conceptual diagram conceptually illustrating one example of a variation pattern table related to the pulse rate.
[FIG. 8] FIG. 8 is a conceptual diagram conceptually illustrating one example of a variation pattern table related to the blood flow volume.
[FIG. 9] FIG. 9 is a conceptual diagram conceptually illustrating one example of a variation pattern table related to a combination of the pulse rate and the blood flow volume.
[FIG. 10] FIG. 10 is a conceptual diagram conceptually illustrating one example of a variation pattern table related to the pulse rate, the blood flow volume and the pulse wave amplitude.
[FIG. 11] FIG. 11 is a flowchart illustrating a flow of high risk level judgment processing.

### Mode for Carrying Out the Invention

Hereinafter, an embodiment of the present invention will be described.

In order to solve the above subject, a blood pressure decrease prediction apparatus in an embodiment is a blood pressure decrease prediction apparatus which is configured to predict a decrease in blood pressure of a living body, according to independent claim 1.

In operation of the blood pressure decrease prediction apparatus in the embodiment, firstly, the pulse rate of the living body is calculated by the pulse rate calculating device, and the blood flow volume of the living body is calculated by the blood flow volume calculating device. The pulse rate calculating device and the blood flow volume calculating device may use different measuring instruments or may use the same measuring instrument to calculate the pulse rate and the blood flow volume, respectively. For example, if different measuring instruments are used, an electrocardiograph can be used to calculate the pulse rate, and a blood flow meter can be used to obtain the blood flow volume. On the other hand, if the same measuring instrument is used, for example, only the blood flow meter can be used to calculate the pulse rate and the blood flow volume. More specifically, a blood flow waveform (i.e. a waveform signal which represents a temporal variation in the blood flow volume) of the living body who is an analyte or a patient is inputted from a laser blood flow meter, which uses, for example, a LDF (Laser Doppler Flowmetry) method, and the pulse rate and the blood flow volume of the living body are calculated by the pulse rate calculating device and the blood flow volume calculating device, respectively, on the basis of the inputted blood flow waveform. The pulse rate calculating device typically calculates, as the pulse rate, an oscillation frequency (i.e. an inverse number of a cycle) corresponding to a pulse wave of the blood flow waveform. The blood flow volume calculating device typically calculates, as the blood flow volume, an average value of the blood flow waveform during a predetermined time period.

Particularly in the embodiment, the risk level which indicates a risk of occurrence of the decrease in blood pressure (i.e. a possibility of occurrence of the decrease in blood pressure) is determined by the risk level determining device on the basis of the variation in at least one of the calculated pulse rate and the calculated blood flow volume. The risk level determining device determines the risk level by referring to a predetermined risk level determination table in which a combination of the variation in the pulse rate and the variation in the blood flow volume is associated with the risk level.

The risk level determining device may use either one of the calculated pulse rate and the calculated blood flow volume or may use both of the calculated pulse rate and the calculated blood flow volume to determine the risk level. In the case where both of the calculated pulse rate and the calculated blood flow volume are used to determine the risk level, the risk level can be determined, more accurately, in comparison with the case where either one of the calculated pulse rate and the calculated blood flow volume is used to determine the risk level.

Specifically, for example, the risk level determining device determines that the risk level is "low" if the pulse rate increases or if the blood flow volume decreases. For example, the risk level determining device determines that the risk level is "middle" if the pulse rate becomes constant after increasing or if the blood flow volume becomes constant after decreasing. For example, the risk level determining device determines that the risk level is "high" if either one of the pulse rate and the blood flow volume becomes constant after decreasing and then further decreases. Alternatively, the risk level determining device determines the risk level by using the risk level determination table or the like which stores therein the value of the risk level in association with the combination of the variation in the pulse rate and the variation in the blood flow volume. In this manner, the risk level determining device can determine the risk level, more accurately, in accordance with the variations in both of the pulse rate and the blood flow volume. Incidentally, the risk level determining device may use another parameter (e.g. pulse wave amplitude, etc.) in addition to the pulse rate and the blood flow volume, to determine the risk level.

Here, the blood pressure is determined on the basis of the product of a beat volume (cardiac output, heart output) and a peripheral vascular resistance (PVR). The "beat volume" is a volume of the blood pumped (transferred) from the heart per minute and varies depending on a heart rate (a cardiac rate) which varies due to the operation of an autonomic nervous system (a sympathetic nervous / parasympathetic nervous system). The beat volume is determined on the basis of the product of a one-time beat volume, which is a volume of the blood pumped from the heart per pumping, and the heart rate. Therefore, the blood pressure is determined on the basis of the product of the heart rate, the one-time beat volume and the peripheral vascular resistance. The "peripheral vascular resistance" is a resistance to the flow of the blood in the peripheral artery and varies due to the operation of the autonomic nervous system (especially, the sympathetic nervous system). If the living body is healthy, usually, the living body automatically adjusts the heart rate and the peripheral vascular resistance in order to maintain the blood pressure. In other words, the living body maintains the blood pressure by automatically adjusting the heart rate and the peripheral vascular resistance, if the blood pressure is about to decrease. Therefore, the heart rate and the peripheral vascular resistance often vary before the blood pressure suddenly decreases.

As described above, the risk level determining device determines the risk level of the decrease in blood pressure of the living body on the basis of the variation in at least one of the calculated pulse rate and the calculated blood flow volume. Here, it is considered that the pulse rate corresponds to the heart rate and the blood flow volume often varies depending on a variation in the peripheral vascular resistance. Thus, the risk level determining device can appropriately determine the risk level which indicates the risk of occurrence of the decrease in blood pressure on the basis of the variation in at least one of the pulse rate and the blood flow volume.

In the embodiment, moreover, in addition to the determination of the risk level described above, it is judged by the high risk level judging device whether or not it is in the high risk level state. Specifically, the high risk level judging device judges that it is in the high risk level state in which the risk is higher than the determined risk level, if the variation range of at least one of the calculated pulse rate and the calculated blood flow volume during the predetermined period exceeds the threshold value which is determined in accordance with the determined risk level. The "predetermined period" herein is a period which is set to detect relatively-short-term variations in the pulse rate and the blood flow volume, and is set shorter than a period in which the risk level determining deice detects the variations in the pulse rate and the blood flow volume. The "threshold value which is determined in accordance with the determined risk level" is a threshold value which is determined in accordance with the extent of the risk level determined by the risk level determining device, and is determined to be a lower value as the risk level increases. In other words, as the risk level determined by the risk level determining device increases, it is more easily judged that it is in the high risk level state.

The high risk level judging device may use either one of the calculated pulse rate and the calculated blood flow volume or may use both of the calculated pulse rate and the calculated blood flow volume to judge whether or not it is in the high risk level state. In the case where both of the calculated pulse rate and the calculated blood flow volume are used to judge whether or not it is in the high risk level state, it can be judged whether or not it is in the high risk level state, more accurately, in comparison with the case where either one of the calculated pulse rate and the calculated blood flow volume is used to judge whether or not it is in the high risk level state.

If either one of the calculated pulse rate and the calculated blood flow volume is used to determine the risk level, the other one, which is different from the one used for the determination of the risk level, may be used to judge whether or not it is in the high risk level state. In other words, if the risk level determining device uses the calculated pulse rate to determine the risk level, the high risk level judging device may use the calculated blood flow volume to judge whether or not it is in the high risk level state. In the same manner, if the risk level determining device uses the calculated blood flow volume to determine the risk level, the high risk level judging device may use the calculated pulse rate to judge whether or not it is in the high risk level state.

The study of the present inventors has revealed that although the risk level of the decrease in blood pressure is considered to increase if the pulse rate and the blood flow volume suddenly vary in a short period, the extent of the increase corresponds to the risk level determined by the risk level determining device. For example, if the pulse rate and the blood flow volume suddenly vary in a short period in a relatively high risk level state, the risk level of the decrease in blood pressure relatively increases at a relatively high increasing rate. On the other hand, if the pulse rate and the blood flow volume suddenly vary in a short period in a relatively low risk level state, the risk level of the decrease in blood pressure relatively increases at a relatively low increasing rate.

As described above, by using the variation in at least one of the calculated pulse rate and the calculated blood flow volume during the predetermined period to judge whether or not it is in the high risk level state, it is possible to predict the risk of occurrence of the decrease in blood pressure, more accurately, in comparison with the case where the risk level is determined only by the risk level determining device.

In the embodiment, the risk level determined by the risk level determining device, or the high risk level state judged by the high risk level judging device are outputted to the exterior, for example, as color(s), number(s), character(s), sentence(s), diagram(s), sign(s), sound or the like. This makes it possible to inform the living body who is the analyte or the patient and an involved person who is a doctor or a nurse, of the risk of the decrease in blood pressure. As a result, it is possible to reduce or prevent a delay of a treatment for the living body whose blood pressure is predicted to decrease (i.e. it is possible to treat the living body who has the risk of the decrease in blood pressure, at an early stage).

Incidentally, the risk level and the high risk level state may not be simply outputted, as described above, but may be used for the control of another connected apparatus or similar actions. For example, if it is configured to control the operation of a dialysis apparatus on the basis of the risk level and the high risk level state while the living body who is the patient receives the artificial dialysis, it is possible to preferably reduce the risk of the decrease in blood pressure. Moreover, if another apparatus such as the dialysis apparatus is connected, it is also possible to output the risk level and the high risk level state by reflecting information obtained from the another apparatus. For example, if the dialysis is not actually performed even though signals are received to calculate the pulse rate and the blood flow volume, which are parameters for determining the risk level, it is possible to perform such control that the risk level and the high risk level state are not outputted, after receiving information which indicates that the dialysis is not performed from the dialysis apparatus.

Furthermore, especially in the embodiment, there is such an advantageous effect that little burden or no burden is caused on the living body, in comparison with a case where the blood pressure is measured, for example, by using a cuff or an electrode, because the pulse rate and the blood flow volume are calculated, for example, on the basis of the blood flow waveform inputted from the laser blood flow meter.

As explained above, according to the blood pressure decrease prediction apparatus in the embodiment, it is possible to predict the decrease in blood pressure of the living body, at an early stage and with high accuracy, with causing little burden on the living body

In one aspect of the blood pressure decrease prediction apparatus in the embodiment, the high risk level judging device judges that it is in the high risk level state in which the risk of occurrence of the decrease in blood pressure is high, in a case where the variation ranges of both of the calculated pulse rate and the calculated blood flow volume exceed the threshold value which is determined in accordance with the determined risk level, in comparison with a case where the variation range of at least one of the calculated pulse rate and the calculated blood flow volume exceeds the threshold value which is determined in accordance with the determined risk level.

In this aspect, if the variation range of either one of the calculated pulse rate and the calculated blood flow volume exceeds the threshold value which is determined in accordance with the determined risk level, it is judged that it is in a state in which the risk of occurrence of the decrease in blood pressure is relatively low, out of the high risk level state (hereinafter referred to as in a "high risk level A state" as occasion demands)

On the other and, if the variation ranges of both of the calculated pulse rate and the calculated blood flow volume exceed the threshold value which is determined in accordance with the determined risk level, it is judged that it is in a state in which the risk of occurrence of the decrease in blood pressure is relatively high, out of the high risk level state (hereinafter referred to as in a "high risk level B state" as occasion demands)

As described above, it is possible to predict the risk of the decrease in blood pressure, more accurately, by judging whether the risk of occurrence of the decrease in blood pressure is in the high risk level A state or in the high risk level B state, on the basis of which parameter of the calculated pulse rate and the calculated blood flow volume exceeds the threshold value.

In another aspect of the above blood pressure decrease prediction apparatus in the embodiment, the risk level determining device determines the risk level by referring to a predetermined risk level determination table in which the variation in at least one of the pulse rate and the blood flow volume is associated with the risk level.

According to this aspect, the risk level determining device determines the risk level which indicates the risk of occurrence of the decrease in blood pressure by referring to the predetermined risk level determination table. The risk level determination table is a predetermined reference table (or look-up table) which is for determining the risk level and in which the variation in at least one of the pulse rate and the blood flow volume is associated with the risk level when there is the variation.

For example, the risk level determination table stores therein the value of the risk level in association with at least one of variation values of the pulse rate and the blood flow volume. Specifically, the risk level determination table stores therein the risk level "low" in association with the case where the pulse rate increases or the case where the blood flow volume decreases, the risk level "middle" in association with the case where the pulse rate becomes constant after increasing or the case where the blood flow volume becomes constant after decreasing, and the risk level "high" in association with the case where either one of the pulse rate and the blood flow volume becomes constant after decreasing and then further decreases.

Alternatively, the risk level determination table stores therein the value of the risk level in association with the combination of the variation in the pulse rate and the variation in the blood flow volume. Specifically, as the risk level when the pulse rate increases and the blood flow volume becomes constant, "LV1" is stored. As the risk level when the pulse rate becomes constant and the blood flow volume decreases, "LV1" is stored. As the risk level when the pulse rate increases and the blood flow volume decreases, there is stored "LV2" which indicates that there is a higher risk of occurrence of the decrease in blood pressure than "LV1". As the risk level when the pulse rate becomes constant after increasing and the blood flow volume decreases, there is stored "LV3" which indicates that there is a higher risk of occurrence of the decrease in blood pressure than "LV2". As the risk level when the pulse rate increases and the blood flow volume becomes constant after decreasing, "LV3" is stored. As the risk level when the pulse rate becomes constant after increasing and the blood flow volume becomes constant after decreasing, there is stored "LV4" which indicates that there is a higher risk of occurrence of the decrease in blood pressure than "LV3". As the risk level when the pulse rate becomes constant after increasing and then further decreases and the blood flow volume becomes constant after decreasing, there is stored "LV5" which indicates that there is a higher risk of occurrence of the decrease in blood pressure than "LV4". As the risk level when the pulse rate becomes constant after increasing and the blood flow volume becomes constant after decreasing and then further decreases, "LV5" is stored. As the risk level when the pulse rate becomes constant after increasing and then further decreases and the blood flow volume becomes constant after decreasing and then further decreases, there is stored "LV6" which indicates that there is a higher risk of occurrence of the decrease in blood pressure than "LV5".

The risk level determination table may store therein the value of the risk level in association with a combination of another parameter (e.g. pulse wave amplitude, etc.) and the variations in the pulse rate and the blood flow volume.

Incidentally, the risk level determination table can be prepared by presuming, experimentally, experientially or by a simulation in advance, the risk of occurrence of the decrease in blood pressure in the case where there are the variation in the pulse rate and the variation in the blood flow volume.

If the variation values of the calculated pulse rate and the calculated blood flow volume match any of the variation values of the pulse rate and the blood flow volume stored in the risk level determination table, the risk level determining device determines the risk level to be one that is associated with the matched combination. Incidentally, if the variation values of the calculated pulse rate and the calculated blood flow volume do not match all of the variation values of the pulse rate and the blood flow volume stored in the risk level determination table, for example, the risk level may be determined to be "none" which indicates that there is little risk or an extremely low risk.

In this aspect, the use of the risk level determination table allows the risk level to be determined relatively easily and accurately. Therefore, it is possible to more preferably predict the decrease in blood pressure of the living body and to more appropriately determine the risk level which indicates the risk of occurrence of the decrease in blood pressure.

In another aspect of the blood pressure decrease prediction apparatus in the embodiment, the pulse rate calculating device calculates the pulse rate of the living body on the basis of a blood flow waveform which represents a temporal variation in the blood flow volume of the living body, and the blood flow volume calculating device calculates the blood flow volume of the living body on the basis of the blood flow waveform.

According to this aspect, each of the pulse rate and the blood flow volume of the living body is calculated from the blood flow waveform which represents the temporal variation in the blood flow volume of the living body. In other words, the pulse rate and the blood flow volume of the living body are calculated not on the basis of different parameters but from the same parameter.

In this aspect, both the two types of parameters, which are the pulse rate and the blood flow volume of the living body and which are used for the determination of the risk level and for the judgment of whether or not it is in the high risk level state, can be calculated from the blood flow waveform which is obtained from, for example, the blood flow meter. It is thus possible to simplify an apparatus configuration and each parameter calculation processing.

In another aspect of the blood pressure decrease prediction apparatus of the first embodiment, further comprising a pulse wave amplitude calculating device which is configured to calculate pulse wave amplitude of the living body, the risk level determining device determining the risk level which indicates the risk of occurrence of the decrease in blood pressure, on the basis of a variation in the calculated pulse wave amplitude, in addition to the variation in at least one of the calculated pulse rate and the calculated blood flow volume.

According to this aspect, it is possible to predict the decrease in blood pressure, at an earlier stage and more appropriately, because the risk level determining device predicts the decrease in blood pressure of the living body on the basis of the variation in the calculated pulse wave amplitude in addition to the variation in at least one of the calculated pulse rate and the calculated blood flow volume.

In an aspect of the blood pressure decrease prediction apparatus which is provided with the pulse wave amplitude calculating device, the risk level determining device determines the risk level by referring to a predetermined risk level determination table in which a combination of the variation in at least one of the pulse rate and the blood flow volume and the variation in the pulse wave amplitude is associated with the risk level.

In this case, the risk level determining device determines the risk level which indicates the risk of occurrence of the decrease in blood pressure by referring to the predetermined risk level determination table. The risk level determination table is a predetermined reference table (or look-up table) which is for determining the risk level and in which the combination of the variation in at least one of the pulse rate and the blood flow volume and the variation in the pulse wave amplitude is associated with the risk level when there is the combination.

For example, in the risk level determination table, as the risk level when the pulse rate becomes constant, the pulse wave amplitude decreases and the blood flow volume becomes constant, "LV1" is stored. As the risk level when the pulse rate increases, the pulse wave amplitude decreases and the blood flow volume becomes constant, "LV2" is stored. As the risk level when the pulse rate becomes constant, the pulse wave amplitude decreases and the blood flow volume decreases, "LV2" is stored. As the risk level when the pulse rate increases, the pulse wave amplitude decreases and the blood flow volume decreases, there is stored "LV3" which indicates that there is a higher risk of occurrence of the decrease in blood pressure than "LV2". As the risk level when the pulse rate becomes constant after increasing, the pulse wave amplitude decreases and the blood flow volume decreases, there is stored "LV4" which indicates that there is a higher risk of occurrence of the decrease in blood pressure than "LV3". As the risk level when the pulse rate increases, the pulse wave amplitude decreases and the blood flow volume becomes constant after decreasing, "LV4" is stored. As the risk level when the pulse rate becomes constant after increasing, the pulse wave amplitude decreases and the blood flow volume becomes constant after decreasing, there is stored "LV5" which indicates that there is a higher risk of occurrence of the decrease in blood pressure than "LV4". As the risk level when the pulse rate becomes constant after increasing and then further decreases, the pulse wave amplitude decreases, and the blood flow volume becomes constant after decreasing, there is stored "LV6" which indicates that there is a higher risk of occurrence of the decrease in blood pressure than "LV5". As the risk level when the pulse rate becomes constant after increasing, the pulse wave amplitude decreases, and the blood flow volume becomes constant after decreasing and then further decreases, "LV6" is stored. As the risk level when the pulse rate becomes constant after increasing and then further decreases, the pulse wave amplitude decreases, and the blood flow volume becomes constant after decreasing and then further decreases, there is stored "LV7" which indicates that there is a higher risk of occurrence of the decrease in blood pressure than "LV6".

If the combination of the variation in the calculated pulse rate, the variation in the calculated blood flow volume and the variation in the calculated pulse wave amplitude matches the combination of the variation in the pulse rate, the variation in the blood flow volume and the variation in the pulse wave amplitude stored in the risk level determination table, the risk level determining device determines the risk level to be one that is associated with the matched combination. Incidentally, if the combination of the variation in the calculated pulse rate, the variation in the calculated blood flow volume and the variation in the calculated pulse wave amplitude does not match all of the combination of the variation in the pulse rate, the variation in the blood flow volume and the variation in the pulse wave amplitude stored in the risk level determination table, the risk level may be determined to be "none" which indicates that there is little risk or an extremely low risk.

In this aspect, the use of the risk level determination table allows the risk level to be determined relatively easily and accurately. Therefore, it is possible to more preferably predict the decrease in blood pressure of the living body and to more appropriately determine the risk level which indicates the risk of occurrence of the decrease in blood pressure.

In an aspect of the blood pressure decrease prediction apparatus which is further provided with the pulse wave amplitude calculating device, the pulse rate calculating device calculates the pulse rate of the living body on the basis of a blood flow waveform which represents a temporal variation in the blood flow volume of the living body, the blood flow volume calculating device calculates the blood flow volume of the living body on the basis of the blood flow waveform, and the pulse wave amplitude calculating device calculates the pulse wave amplitude of the living body on the basis of the blood flow waveform.

In this case, the pulse rate, each of the blood flow volume and the pulse wave amplitude of the living body is calculated from the blood flow waveform which represents the temporal variation in the blood flow volume of the living body. In other words, the pulse rate, the blood flow volume and the pulse wave amplitude of the living body are calculated not on the basis of different parameters but from the same parameter.

In this aspect, all the three types of parameters, which are the pulse rate, the blood flow volume and the pulse wave amplitude of the living body and which are used for the determination of the risk level and for the judgment of whether or not it is in the high risk level state, can be calculated from the blood flow waveform which is obtained from, for example, the blood flow meter. It is thus possible to simplify the apparatus configuration and each parameter calculation processing.

In another aspect of the blood pressure decrease prediction apparatus in the embodiment, further comprising a first outputting device which is configured to output to the exterior the risk level determined by the risk level determining device.

According to this aspect, the outputting device outputs to the exterior the risk level determined by the risk level determining device, for example, as color(s), number(s), character(s), sentence(s), diagram(s), sign(s), sound or the like. Therefore, it is possible to inform the living body who is the analyte or the patient and the involved person who is the doctor or the nurse of the risk, of the decrease in blood pressure. As a result, it is possible to reduce or prevent the delay of the treatment for the living body whose blood pressure is predicted to decrease (i.e. it is possible to treat the living body who has the risk of the decrease in blood pressure, at an early stage).

In another aspect of the blood pressure decrease prediction apparatus in the embodiment, further comprising a second outputting device which is configured to output to the exterior the high risk level state determined by the high risk level judging device.

According to this aspect, the outputting device outputs to the exterior a result of the judgment by the high risk level judging device (i.e. whether or not it is in the high risk level state), for example, as color(s), number(s), character(s), sentence(s), diagram(s), sign(s), sound or the like. Therefore, it is possible to inform the living body who is the analyte or the patient and the involved person who is the doctor or the nurse of the risk, of the decrease in blood pressure. As a result, it is possible to reduce or prevent the delay of the treatment for the living body whose blood pressure is predicted to decrease (i.e. it is possible to treat the living body who has the risk of the decrease in blood pressure, at an early stage).

These operations and other advantages of the present invention will become more apparent from an example explained below.

### Example

Hereinafter, an example of the present invention will be described with reference to the drawings.

### <Apparatus Configuration>

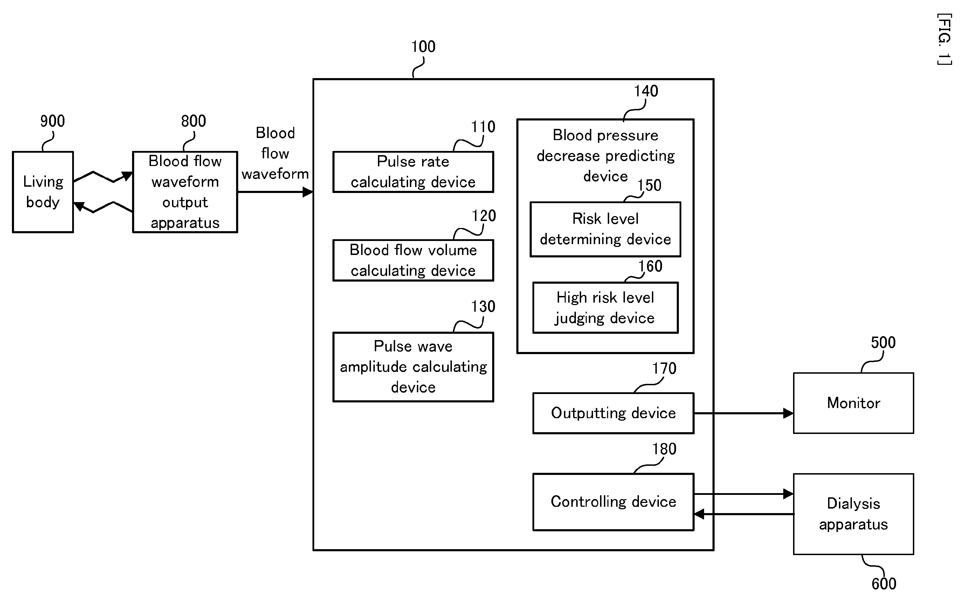

Firstly, a configuration of a blood pressure decrease prediction apparatus in the example will be explained with reference to FIG. 1. FIG. 1 is a block diagram illustrating the configuration of the blood pressure decrease prediction apparatus in the example.

In FIG. 1, the blood pressure decrease prediction apparatus 100 in the example is an apparatus for predicting a decrease in blood pressure of a living body 900 while the living body 900, who is a patient, receives artificial dialysis using a dialysis apparatus 600. The blood pressure decrease prediction apparatus 100 is provided with a pulse rate calculating device 110, a blood flow volume calculating device 120, a pulse wave amplitude calculating device 130, a blood pressure decrease predicting device 140 which includes a risk level determining device 150 and a high risk level judging device 160, and the outputting device 170.

The blood pressure decrease prediction apparatus 100 is configured such that a blood flow waveform which is outputted from a blood flow waveform output apparatus 800 is inputted thereto. The blood flow waveform output apparatus 800 is, for example, a laser blood flow meter using a LDF (Laser Doppler Flowmetry) method, and outputs the blood flow waveform of the living body 900 (i.e. a waveform signal which represents a temporal variation in blood flow volume).

The pulse rate calculating device 110, the blood flow volume calculating device 120 and the pulse wave amplitude calculating device 130 calculate a pulse rate, a blood flow volume and pulse wave amplitude of the living body 900, respectively, on the basis of the blood flow waveform inputted from the blood flow waveform output apparatus 800.

The risk level determining device 150 determines a risk level which indicates a risk of occurrence of the decrease in blood pressure of the living body 900, by using variations in the pulse rate, the blood flow volume and the pulse wave amplitude of the living body 900, which are calculated by the pulse rate calculating device 110, the blood flow volume calculating device 120 and the pulse wave amplitude calculating device 130, respectively.

The risk level judging device judges whether or not it is in a high risk level state in which the risk of occurrence of the decrease in blood pressure of the living body 900 is extremely high, by using short-term variations in the pulse rate, the blood flow volume and the pulse wave amplitude of the living body 900, which are calculated by the pulse rate calculating device 110, the blood flow volume calculating device 120 and the pulse wave amplitude calculating device 130, respectively.

The outputting device 170 outputs the risk level determined by the risk level determining device 150 and a result of the judgment of the high risk level judging device 160 (i.e. whether or not it is in the high risk level state), to a monitor.

A controlling device 180 controls the operation of the dialysis apparatus 600 in accordance with the risk level determined by the risk level determining device 150 and the result of the judgment of the high risk level judging device 160. The controlling device 180 is also configured to receive various information 600 (e.g. an operating state of the dialysis apparatus, various parameters during the dialysis, etc.) from the dialysis apparatus.

### <Parameter Calculation Processing>

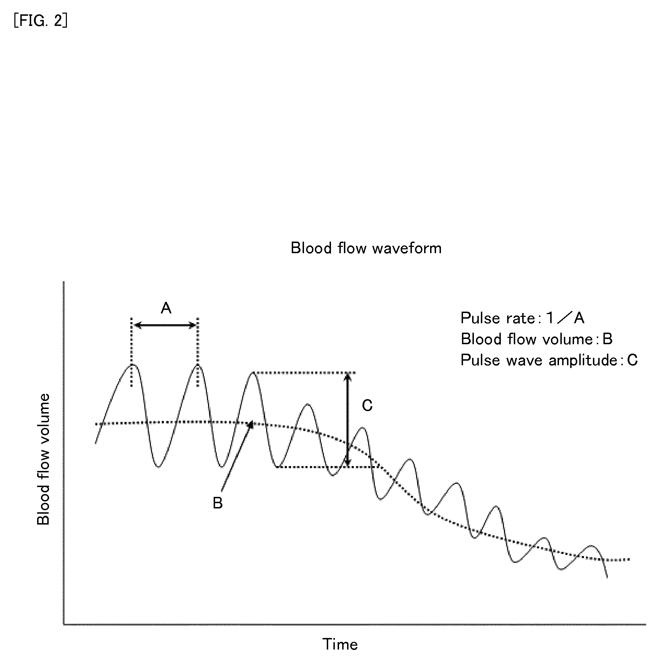

Next, a specific method of calculating the pulse rate, the blood flow volume and the pulse wave amplitude on the pulse rate calculating device 110, the blood flow volume calculating device 120 and the pulse wave amplitude calculating device 130 will be explained with reference to FIG. 2. FIG. 2 is an explanatory diagram for explaining one example of the method of calculating the pulse rate, the blood flow volume and the pulse wave amplitude, based on the blood flow waveform. Incidentally, FIG. 2 illustrates one example of the blood flow waveform which is outputted from the blood flow waveform output apparatus 800.

In FIG. 2, the pulse rate calculating device 110 calculates, as the pulse rate, an oscillation frequency of a waveform corresponding to a pulse wave of the blood flow waveform, i.e. an inverse number (1/A) of a cycle A of the blood flow waveform. Incidentally, another calculation method such as a Fast Fourier Transform (FFT) may be used as a method of calculating the pulse rate.

The blood flow volume calculating device 120 calculates, as the blood flow volume, an average value B of the blood flow waveform during a predetermined time period.

The pulse wave amplitude calculating device 130 calculates, as the pulse wave amplitude, an amplitude C of the waveform corresponding to the pulse wave of the blood flow waveform. Incidentally, another calculation method such as a Fast Fourier Transform (FFT) may be used as a method of calculating the pulse wave amplitude.

As described above, the blood pressure decrease prediction apparatus 100 is configured to obtain three variable parameters such as the pulse rate, the blood flow volume and the pulse wave amplitude, from the blood flow waveform which is outputted from the blood flow waveform output apparatus 800.

### <Blood Pressure Decrease Prediction Processing>

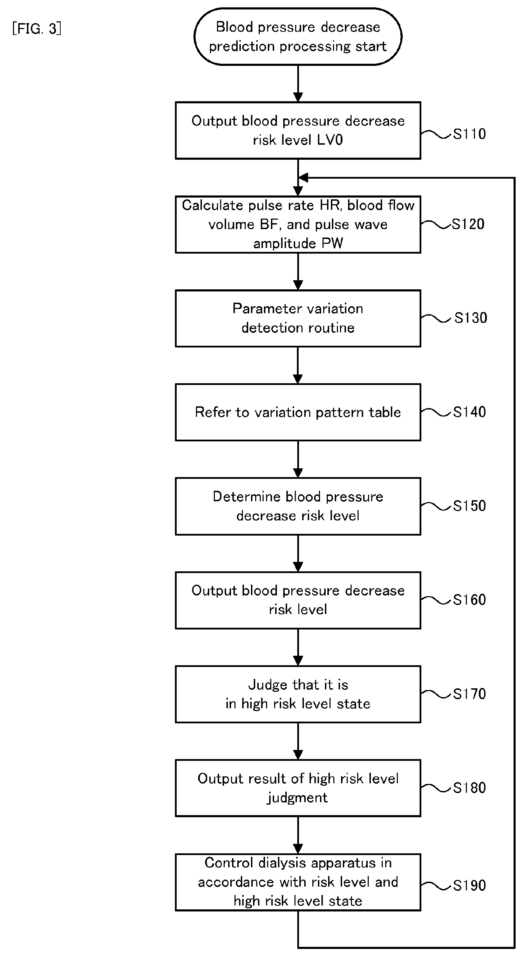

Next, blood pressure decrease prediction processing which is performed by the blood pressure decrease predicting device 140 will be explained with reference to FIG. 3. FIG. 3 is a flowchart illustrating a flow of the blood pressure decrease prediction processing.

In FIG. 3, if the blood pressure decrease prediction processing is started, firstly, "LV0" is outputted by the outputting device 170 (step S110). Namely, the risk level determining device 150 sets, as an initial value, a blood pressure decrease risk level at "LV0" which indicates the risk of the decrease in blood pressure is little or very low, and the outputting device 170 outputs the set blood pressure decrease risk level "LV0".

Then, a pulse rate HR, a blood flow volume BF and a pulse wave amplitude PW are calculated on the pulse rate calculating device 110, the blood flow volume calculating device 120 and the pulse wave amplitude calculating device 130, respectively (step S120). The pulse rate calculating device 110, the blood flow volume calculating device 120 and the pulse wave amplitude calculating device 130 calculate the pulse rate HR, the blood flow volume BF and the pulse wave amplitude PW, respectively, on the basis of the blood flow waveform, as described above.

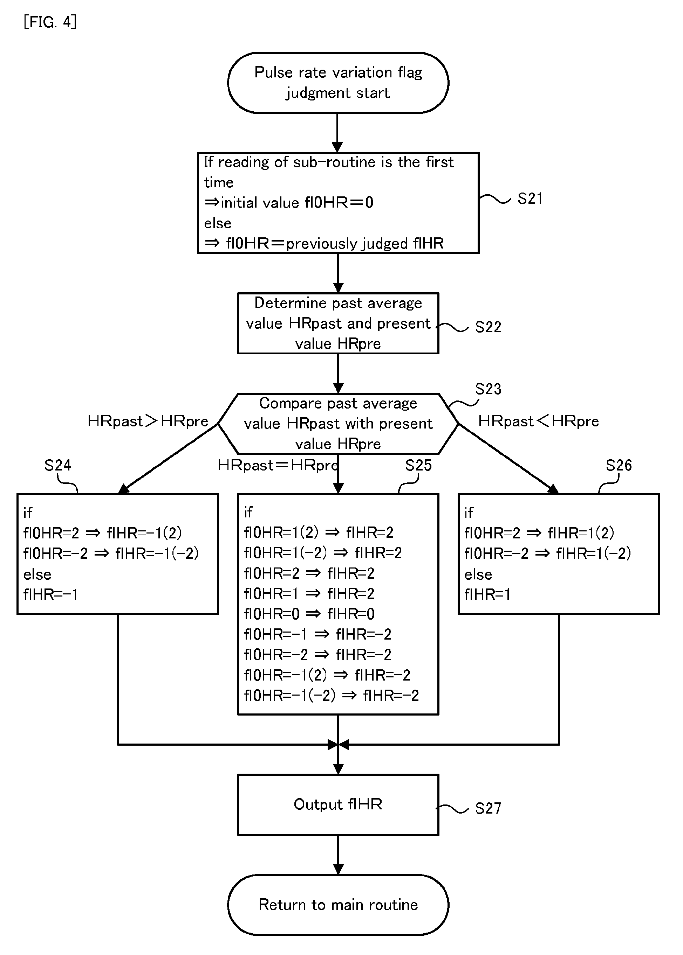

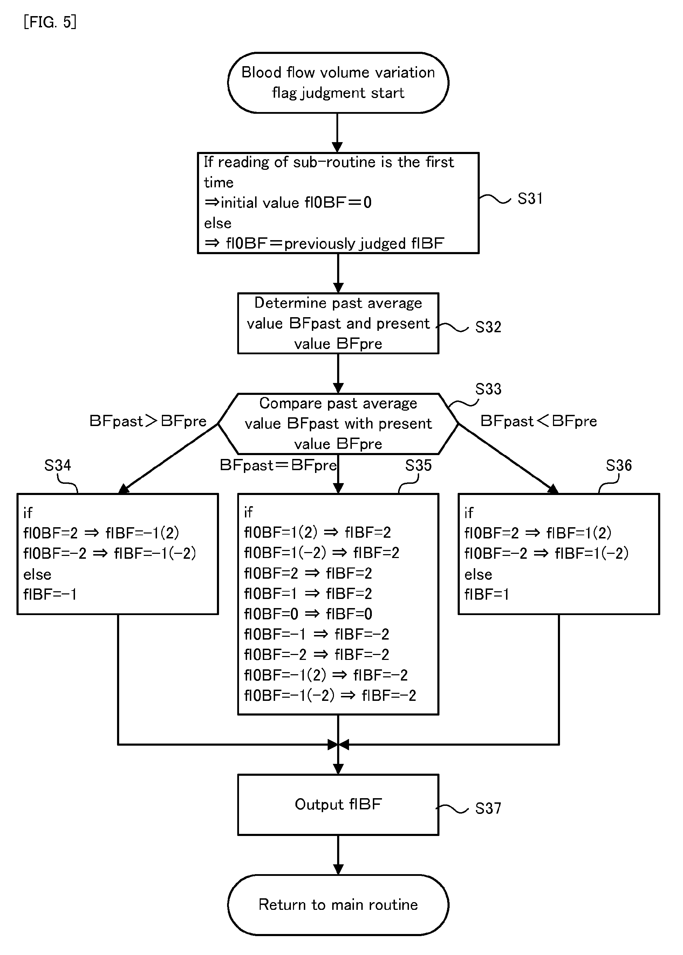

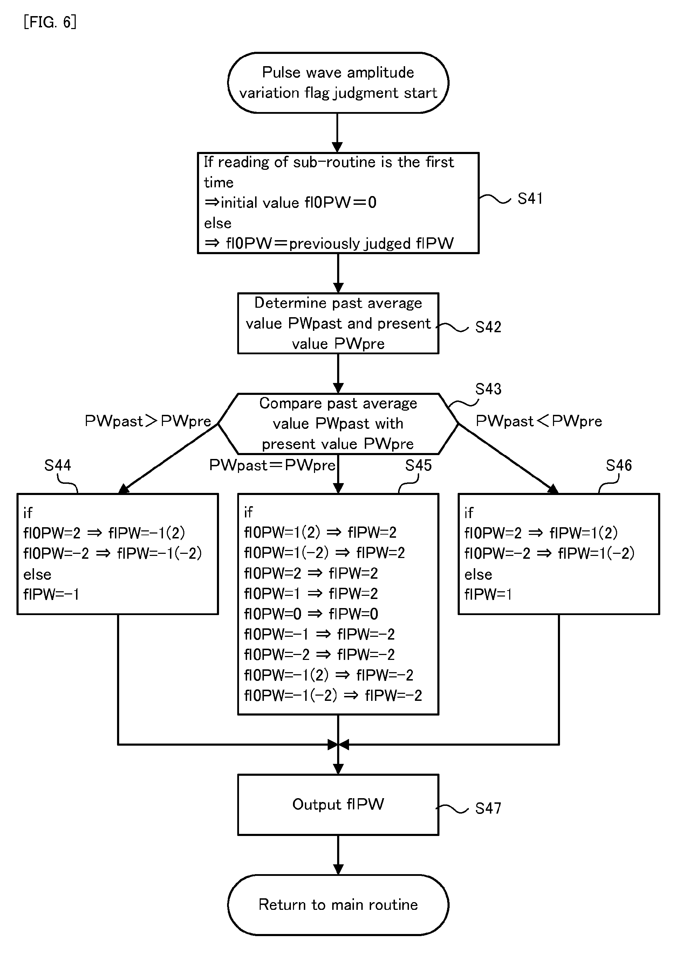

If the pulse rate HR, the blood flow volume BF and the pulse wave amplitude PW are calculated, sub-routines for detecting respective variations in the pulse rate HR, the blood flow volume BF and the pulse wave amplitude PW are started (step S130). Hereinafter, the variation detection sub-routine will be explained in detail with reference to FIG. 4 to FIG. 6. FIG. 4 is a flowchart illustrating a flow of processing in the variation detection sub-routine for the pulse rate. FIG. 5 is a flowchart illustrating a flow of processing in the variation detection sub-routine for the blood flow volume. FIG. 6 is a flowchart illustrating a flow of processing in the variation detection sub-routine for the pulse wave amplitude.

The variations in the pulse rate HR, the blood flow volume BF and the pulse wave amplitude PW are detected by judging a pulse rate variation flag flHR which indicates the variation in the pulse rate HR, a blood flow volume variation flag flBF which indicates the variation in the blood flow volume BF, and a pulse wave amplitude variation flag flPW which indicates the variation in the pulse wave amplitude PW. Each of the pulse rate variation flag flHR, the blood flow volume variation flag flBF and the pulse wave amplitude variation flag flPW can take values of "0" which indicates no change, "1" which indicates an increase, "-1" which indicates a decrease, "2" which indicates becoming constant after increasing, and "-2" which indicates becoming constant after decreasing. Moreover, "1(2)" denotes becoming constant after increasing (i.e. becoming in a state of "2") and then increasing, "-1(2)" denotes becoming constant after increasing (i.e. becoming in a state of "2") and then decreasing, "1(-2)" denotes becoming constant after increasing (i.e. becoming in a state of "-2") and then further increasing, and a "-1(-2)" denotes becoming constant after decreasing (i.e. becoming in a state of "-2") and then further decreasing.

In FIG. 4, in the judgment of the pulse rate variation flag flHR, "0" is firstly set as an initial value fl0HR of the pulse rate variation flag flHR. Incidentally, if the judgment sub-routine of the pulse rate variation flag flHR is not the first time, a value of the pulse rate variation flag flHR which is previously judged is set as the initial value fl0HR (step S21).

Then, a past average value HRpast of the pulse rate HR and a present value HRpre are determined (step S22). The past average value HRpast is calculated, for example, as a past five-minute average value. It is not only the average value but also may be one point such as the past maximum value. The present value HRpre is calculated, for example, as a latest one-minute average value.

If the past average value HRpast and the present value HRpre are determined, the past average value HRpast and the present value HRpre are compared (step S23). Here, if HRpast>HRpre (i.e. if the pulse rate HR decreases), the processing moves to a step S24. If HRpast=HRpre (i.e. if the pulse rate HR does not change), the processing moves to a step S25. If HRpast<HRpre (i.e. if the pulse rate HR increases), the processing moves to a step S26.

In the case where HRpast>HRpre (i.e. if the pulse rate HR decreases), if the initial value fl0HR is "2", the pulse rate variation flag flHR is set at "-1(2)". If the initial value fl0HR is "-2", the pulse rate variation flag flHR is set at "-1(-2)". In the other cases, the pulse rate variation flag flHR is set at "-1" (step S24).

In the case where HRpast=HRpre (i.e. if the pulse rate HR does not change), if the initial value fl0HR is "1(2)", the pulse rate variation flag flHR is set at "2". If the initial value fl0HR is "1(-2)", the pulse rate variation flag flHR is set at "2". If the initial value fl0HR is "2", the pulse rate variation flag flHR is set at "2". If the initial value fl0HR is "1", the pulse rate variation flag flHR is set at "2". If the initial value fl0HR is "0", the pulse rate variation flag flHR is set at "0". If the initial value fl0HR is "-1", the pulse rate variation flag flHR is set at "-2". If the initial value fl0HR is "-2", the pulse rate variation flag flHR is set at "-2". If the initial value fl0HR is "-1(2)", the pulse rate variation flag flHR is set at "-2". If the initial value fl0HR is "-1(-2)", the pulse rate variation flag flHR is set at "-2" (step S25).

In the case where HRpast<HRpre (i.e. if the pulse rate HR increases), if the initial value fl0HR is "2", the pulse rate variation flag flHR is set at "1(2)". If the initial value fl0HR is "-2", the pulse rate variation flag flHR is set at "1(-2)". In the other cases, the pulse rate variation flag flHR is set at "1" (step S26).

As described above, the pulse rate variation flag flHR is determined by case analysis and is outputted (step S27). If the pulse rate variation flag flHR is outputted, the sub-routine is ended to return to the main routine (the processing illustrated in FIG. 3).

In FIG. 5, in the judgment of the blood flow volume variation flag fIBF, "0" is firstly set as an initial value fl0BF of the blood flow volume variation flag flBF. Incidentally, if the judgment sub-routine of the blood flow volume variation flag flBF is not the first time, a value of the blood flow volume variation flag flBF which is previously judged is set as the initial value fl0BF (step S31).

Then, a past average value BFpast of the blood flow volume BF and a present value BFpre are determined (step S32). The past average value BFpast is calculated, for example, as a past five-minute average value. It is not only the average value but also may be one point such as the past maximum value. The present value BFpre is calculated, for example, as a latest one-minute average value.

If the past average value BFpast and the present value BFpre are determined, the past average value BFpast and the present value BFpre are compared (step S33). Here, if BFpast>BFpre (i.e. if the blood flow volume BF decreases), the processing moves to a step S34. If BFpast=BFpre (i.e. if the blood flow volume BF does not change), the processing moves to a step S35. If BFpast<BFpre (i.e. if the blood flow volume BF increases), the processing moves to a step S36.

In the case where BFpast>BFpre (i.e. if the blood flow volume BF decreases), if the initial value fl0BF is "2", the blood flow volume variation flag flBF is set at "-1(2)". If the initial value fl0BF is "-2", the blood flow volume variation flag flBF is set at "-1(-2)". In the other cases, the blood low volume variation flag flBF is set at "-1" (step S34).

In the case where BFpast=BFpre (i.e. if the blood flow volume BF does not change), if the initial value fl0BF is "1(2)", the blood flow volume variation flag flBF is set at "2". If the initial value fl0BF is "1(-2)", the blood flow volume variation flag flBF is set at "2". If the initial value fl0BF is "2", the blood flow volume variation flag flBF is set at "2". If the initial value fl0BF is "1", the blood flow volume variation flag flBF is set at "2". If the initial value fl0BF is "0", the blood flow volume variation flag flBF is set at "0". If the initial value fl0BF is "-1", the blood flow volume variation flag flBF is set at "-2". If the initial value fl0BF is "-2", the blood flow volume variation flag flBF is set at "-2". If the initial value fl0BF is "-1(2)", the blood flow volume variation flag flBF is set at "-2". If the initial value fl0BF is "-1(-2)", the blood flow volume variation flag flBF is set at "-2" (step S35).

In the case where BFpast<BFpre (i.e. if the blood flow volume BF increases), if the initial value fl0BF is "2", the blood flow volume variation flag flBF is set at "1(2)". If the initial value fl0BF is "-2", the blood flow volume variation flag flBF is set at "1(-2)". In the other cases, the blood flow volume variation flag flBF is set at "1" (step S36).

As described above, the blood flow volume variation flag flBF is determined by case analysis and is outputted (step S37). If the blood flow volume variation flag flBF is outputted, the sub-routine is ended to return to the main routine (the processing illustrated in FIG. 3).

In FIG. 6, in the judgment of the pulse wave amplitude variation flag flPW, "0" is firstly set as an initial value fl0PW of the pulse wave amplitude variation flag flPW. Incidentally, if the judgment sub-routine of the pulse wave amplitude variation flag flPW is not the first time, a value of the pulse wave amplitude variation flag flPW which is previously judged is set as the initial value fl0PW (step S41).

Then, a past average value PWpast of the pulse wave amplitude PW and a present value PWpre are determined (step S42). The past average value PWpast is calculated, for example, as a past five-minute average value. It is not only the average value but also may be one point such as the past maximum value. The present value PWpre is calculated, for example, as a latest one-minute average value.

If the past average value PWpast and the present value PWpre are determined, the past average value PWpast and the present value PWpre are compared (step S43). Here, if PWpast>PWpre (i.e. if the pulse wave amplitude PW decreases), the processing moves to a step S44. If PWpast=PWpre (i.e. if the pulse wave amplitude PW does not change), the processing moves to a step S45. If PWpast<PWpre (i.e. if the pulse wave amplitude PW increases), the processing moves to a step S46.

In the case where PWpast>PWpre (i.e. if the pulse wave amplitude PW decreases), if the initial value fl0PW is "2", the pulse wave amplitude variation flag flPW is set at "-1(2)". If the initial value fl0PW is "-2", the pulse wave amplitude variation flag flPW is set at "-1(-2)". In the other cases, the pulse wave amplitude variation flag flPW is set at "-1" (step S44).

In the case where PWpast=PWpre (i.e. if the pulse wave amplitude PW does not change), if the initial value fl0PW is "1(2)", the pulse wave amplitude variation flag flPW is set at "2". If the initial value fl0PW is "1(-2)", the pulse wave amplitude variation flag flPW is set at "2". If the initial value fl0PW is "2", the pulse wave amplitude variation flag flPW is set at "2". If the initial value fl0PW is "1", the pulse wave amplitude variation flag flPW is set at "2". If the initial value fl0PW is "0", the pulse wave amplitude variation flag flPW is set at "0". If the initial value fl0PW is "-1", the pulse wave amplitude variation flag flPW is set at "-2". If the initial value fl0PW is "-2", the pulse wave amplitude variation flag flPW is set at "-2". If the initial value fl0PW is "-1(2)", the pulse wave amplitude variation flag flPW is set at "-2". If the initial value fl0PW is "-1(-2)", the pulse wave amplitude variation flag flPW is set at "-2" (step S45).

In the case where PWpast<PWpre (i.e. if the pulse wave amplitude PW increases), if the initial value fl0PW is "2", the pulse wave amplitude variation flag flPW is set at "1(2)". If the initial value fl0PW is "-2", the pulse wave amplitude variation flag flPW is set at "1(-2)". In the other cases, the pulse wave amplitude variation flag flPW is set at "1" (step S46).

As described above, the pulse wave amplitude variation flag flPW is determined by case analysis and is outputted (step S47). If the pulse wave amplitude variation flag flPW is outputted, the sub-routine is ended to return to the main routine (the processing illustrated in FIG. 3).

Back in FIG. 3, if the pulse rate variation flag flHR, the blood flow volume variation flag flBF and the pulse wave amplitude variation flag flPW are outputted by using the aforementioned sub-routines, the values of the pulse rate variation flag flHR, the blood flow volume variation flag flBF and the pulse wave amplitude variation flag flPW are compared with a preset variation pattern table.

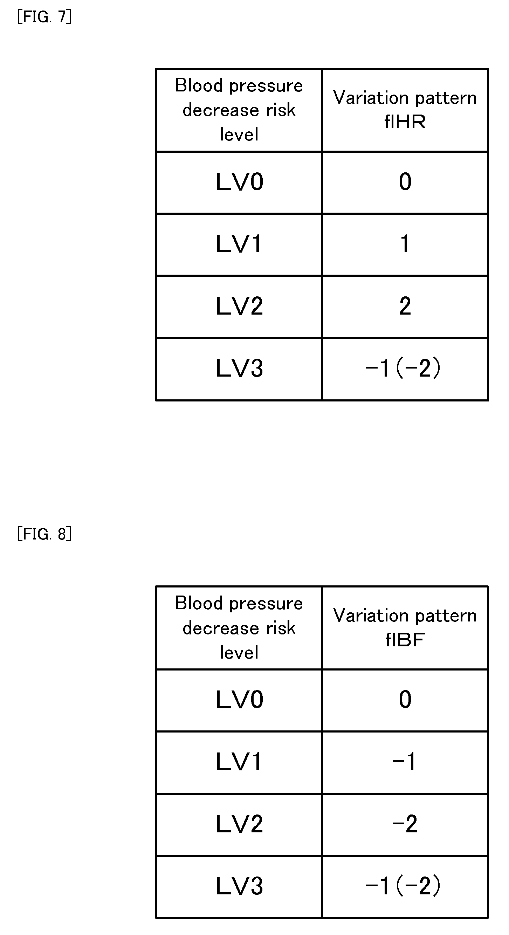

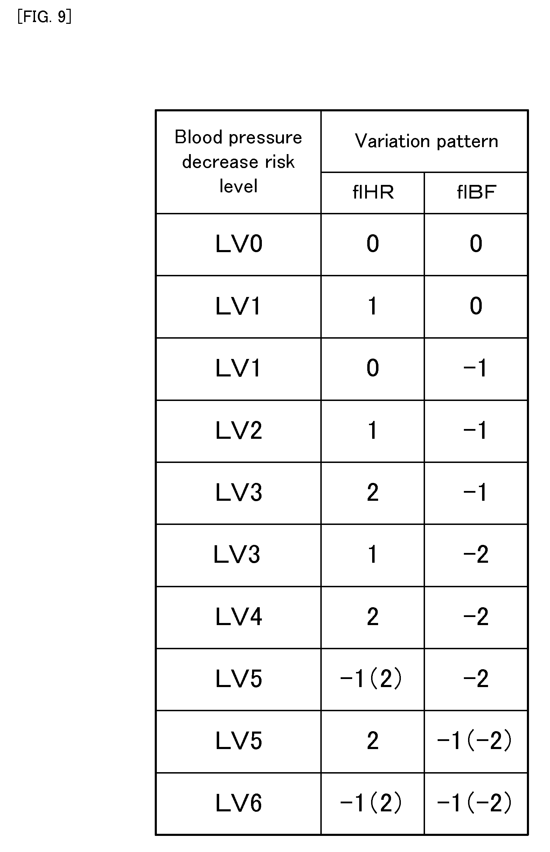

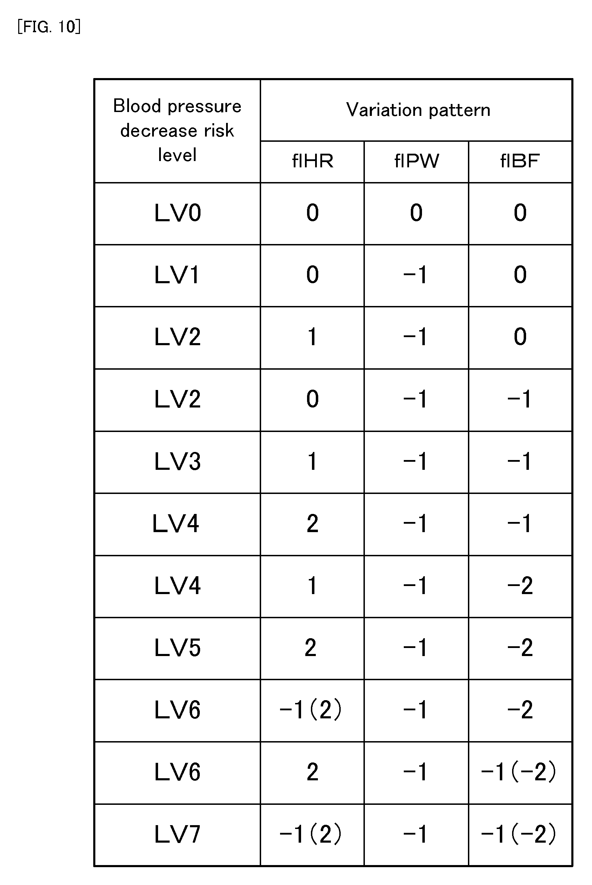

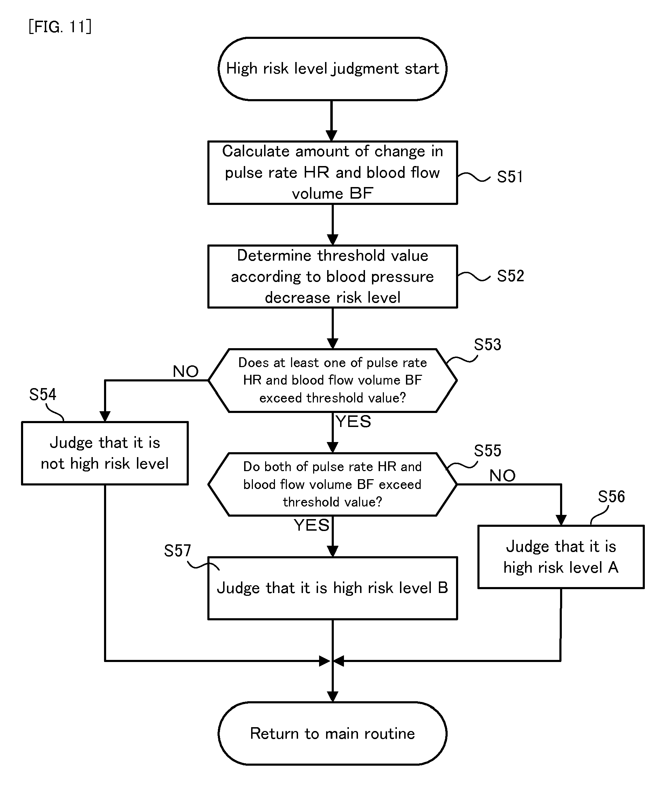

Hereinafter, the variation pattern table will be specifically explained with reference to FIG. 7 to FIG. 10. FIG. 7 is a conceptual diagram conceptually illustrating one example of the variation pattern table related to the pulse rate. FIG. 8 is a conceptual diagram conceptually illustrating one example of the variation pattern table related to the blood low volume. FIG. 9 is a conceptual diagram conceptually illustrating one example of the variation pattern table related to the combination of the pulse rate and the blood flow volume. FIG. 10 is a conceptual diagram conceptually illustrating one example of the variation pattern table related to the pulse rate, the blood flow volume and the pulse wave amplitude. FIG. 11 is a flowchart illustrating a flow of high risk level judgment processing.

In FIG. 7 and FIG. 8, with regard to the values of the pulse rate variation flag flHR, the blood flow volume variation flag flBF and the pulse wave amplitude variation flag flPW, the use of not all the three values but at least one of the pulse rate variation flag flHR and the blood flow volume variation flag flBF can provide considerably accurate prediction of the risk of the decrease in blood pressure.

On the variation pattern table related to the pulse rate HR illustrated in FIG. 7, "LV0" which indicates that there is little or a very low risk of the decrease in blood pressure is stored as the blood pressure decrease risk level when the pulse rate variation flag flHR is "0". As the blood pressure decrease risk level when the pulse rate variation flag flHR is "1", there is stored "LV1" which indicates that there is a higher risk of occurrence of the decrease in blood pressure than "LV0". As the blood pressure decrease risk level when the pulse rate variation flag flHR is "2", there is stored "LV2" which indicates that there is a higher risk of occurrence of the decrease in blood pressure than "LV1". As the blood pressure decrease risk level when the pulse rate variation flag flHR is "-1(-2)", there is stored "LV3" which indicates that there is a higher risk of occurrence of the decrease in blood pressure than "LV2".

On the variation pattern table related to the blood flow volume BF illustrated in FIG. 8, "LV0" which indicates that there is little or a very low risk of the decrease in blood pressure is stored as the blood pressure decrease risk level when the blood flow volume variation flag flBF is "0". As the blood pressure decrease risk level when the blood flow volume variation flag flBF is "-1", there is stored "LV1" which indicates that there is a higher risk of occurrence of the decrease in blood pressure than "LV0". As the blood pressure decrease risk level when the blood flow volume variation flag flBF is "-2", there is stored "LV2" which indicates that there is a higher risk of occurrence of the decrease in blood pressure than "LV1". As the blood pressure decrease risk level when the blood flow volume variation flag flBF is "-1(-2)", there is stored "LV3" which indicates that there is a higher risk of occurrence of the decrease in blood pressure than "LV2".

In FIG. 9, the use of both of the pulse rate variation flag flHR and the blood flow volume variation flag flBF can provide more accurate prediction of the risk of the decrease in blood pressure than the use of at least one of the pulse rate variation flag flHR and the blood flow volume variation flag flBF.

On the variation pattern table related to the combination of the pulse rate and the blood flow volume illustrated in FIG. 9, "LV0" which indicates that there is little or a very low risk of the decrease in blood pressure is stored as the blood pressure decrease risk level when the pulse rate variation flag flHR is "0" and the blood flow volume variation flag flBF is "0". As the blood pressure decrease risk level when the pulse rate variation flag flHR is "1" and the blood flow volume variation flag flBF is "0", there is stored "LV1" which indicates that there is a higher risk of occurrence of the decrease in blood pressure than "LV0". As the blood pressure decrease risk level when the pulse rate variation flag flHR is "0" and the blood flow volume variation flag flBF is "-1", there is stored "LV1" which indicates that there is a higher risk of occurrence of the decrease in blood pressure than "LV0". As the blood pressure decrease risk level when the pulse rate variation flag flHR is "1" and the blood flow volume variation flag flBF is "-1", there is stored "LV2" which indicates that there is a higher risk of occurrence of the decrease in blood pressure than "LV1". As the blood pressure decrease risk level when the pulse rate variation flag flHR is "2" and the blood flow volume variation flag flBF is "-1", there is stored "LV3" which indicates that there is a higher risk of occurrence of the decrease in blood pressure than "LV2". As the blood pressure decrease risk level when the pulse rate variation flag flHR is "1" and the blood flow volume variation flag flBF is "-2", there is stored "LV3" which indicates that there is a higher risk of occurrence of the decrease in blood pressure than "LV2". As the blood pressure decrease risk level when the pulse rate variation flag flHR is "2" and the blood flow volume variation flag flBF is "-2", there is stored "LV4" which indicates that there is a higher risk of occurrence of the decrease in blood pressure than "LV3". As the blood pressure decrease risk level when the pulse rate variation flag flHR is "-1(2)" and the blood flow volume variation flag flBF is "-2", there is stored "LV5" which indicates that there is a higher risk of occurrence of the decrease in blood pressure than "LV4". As the blood pressure decrease risk level when the pulse rate variation flag flHR is "2" and the blood flow volume variation flag flBF is "-1(-2)", there is stored "LV5" which indicates that there is a higher risk of occurrence of the decrease in blood pressure than "LV4". As the blood pressure decrease risk level when the pulse rate variation flag flHR is "-1(2)" and the blood flow volume variation flag flBF is "-1(-2)", there is stored "LV6" which indicates that there is a higher risk of occurrence of the decrease in blood pressure than "LV5".

In FIG. 10, with regard to the values of the pulse rate variation flag flHR, the blood flow volume variation flag flBF and the pulse wave amplitude variation flag flPW, the use of all the three values can provide extremely accurate prediction of the risk of the decrease in blood pressure.

On the variation pattern table related to the pulse rate, the blood flow volume and the pulse wave amplitude illustrated in FIG. 10, "LV0" which indicates that there is little or a very low risk of the decrease in blood pressure is stored as the blood pressure decrease risk level when the pulse rate variation flag flHR is "0", the pulse wave amplitude variation flag flPW is "0" and the blood flow volume variation flag flBF is "0". As the blood pressure decrease risk level when the pulse rate variation flag flHR is "0", the pulse wave amplitude variation flag flPW is "-1" and the blood flow volume variation flag flBF is "0", there is stored "LV1" which indicates that there is a higher risk of occurrence of the decrease in blood pressure than "LV0". As the blood pressure decrease risk level when the pulse rate variation flag flHR is "1", the pulse wave amplitude variation flag flPW is "-1" and the blood flow volume variation flag flBF is "0", there is stored "LV2" which indicates that there is a higher risk of occurrence of the decrease in blood pressure than "LV1". As the blood pressure decrease risk level when the pulse rate variation flag flHR is "0", the pulse wave amplitude variation flag flPW is "-1" and the blood flow volume variation flag flBF is "-1", there is stored "LV2" which indicates that there is a higher risk of occurrence of the decrease in blood pressure than "LV1". As the blood pressure decrease risk level when the pulse rate variation flag flHR is "1", the pulse wave amplitude variation flag flPW is "-1" and the blood flow volume variation flag flBF is "-1", there is stored "LV3" which indicates that there is a higher risk of occurrence of the decrease in blood pressure than "LV2". As the blood pressure decrease risk level when the pulse rate variation flag flHR is "2", the pulse wave amplitude variation flag flPW is "-1" and the blood flow volume variation flag flBF is "-1", there is stored "LV4" which indicates that there is a higher risk of occurrence of the decrease in blood pressure than "LV3". As the blood pressure decrease risk level when the pulse rate variation flag flHR is "1", the pulse wave amplitude variation flag flPW is "-1" and the blood flow volume variation flag flBF is "-2", there is stored "LV4" which indicates that there is a higher risk of occurrence of the decrease in blood pressure than "LV3". As the blood pressure decrease risk level when the pulse rate variation flag flHR is "2", the pulse wave amplitude variation flag flPW is "-1" and the blood flow volume variation flag flBF is "-2", there is stored "LV5" which indicates that there is a higher risk of occurrence of the decrease in blood pressure than "LV4". As the blood pressure decrease risk level when the pulse rate variation flag flHR is "-1(2)", the pulse wave amplitude variation flag flPW is "-1" and the blood flow volume variation flag flBF is "-2", there is stored "LV6" which indicates that there is a higher risk of occurrence of the decrease in blood pressure than "LV5". As the blood pressure decrease risk level when the pulse rate variation flag flHR is "2", the pulse wave amplitude variation flag flPW is "-1" and the blood flow volume variation flag flBF is "-1(-2)", there is stored "LV6" which indicates that there is a higher risk of occurrence of the decrease in blood pressure than "LV5". As the blood pressure decrease risk level when the pulse rate variation flag flHR is "-1(2)", the pulse wave amplitude variation flag flPW is "-1" and the blood flow volume variation flag flBF is "-1(-2)", there is stored "LV7" which indicates that there is a higher risk of occurrence of the decrease in blood pressure than "LV6".

The above described variation pattern table can be prepared in advance by estimating, experimentally, experientially or by a simulation in advance, the risk of occurrence of the decrease in blood pressure in cases where there are combinations of the variations in the pulse rate HR, the blood flow volume BF and the pulse wave amplitude PW.

Back in FIG. 3, the risk level determining device 150 determines the blood pressure decrease risk level using a result of the reference to the variation pattern table (step S150). The determined blood pressure decrease risk level is displayed on a monitor 500 by the outputting device 170 (step S160). This makes it possible to inform the living body 900 who is the patient and an involved person who is a doctor or a nurse, of the risk of the decrease in blood pressure. As a result, it is possible to treat the living body 900 who has the risk of the decrease in blood pressure, at an early stage.

In the embodiment, moreover, after the determination of the blood pressure decrease risk level, it is determined whether or not it is in the high risk level state in which the risk level is extremely high, by using short-term variations in the pulse rate HR, the blood flow volume BF and the pulse wave amplitude PW (step S170).

Hereinafter, the high risk level judgment processing will be explained in detail with reference to FIG. 11. FIG. 11 is a flowchart illustrating a flow of the high risk level judgment processing.

In FIG. 11, in the high risk level judgment processing, firstly, the short-term variations or the short-term amount of change in the pulse rate HR and the short-term amount of change the blood flow volume BF are calculated (step S51). The amount of change here is calculated, for example, as a ratio between the present value and the past average value in the past which is appropriate time interval immediately before the present time. In this case, the past average value calculated, for example, as a past 10-second average value may be used. Moreover, the present value calculated, for example, as the latest one-second average value may be used.

Then, a threshold value for the amount of change in the pulse rate HR and the amount of change in the blood flow volume BF is determined according to the blood pressure decrease risk level determined by the risk level determining device 150 (step S52). The threshold value is determined typically as a lower value as the blood pressure decrease risk level increases. In other words, the threshold value is set to be more easily exceeded as the blood pressure decrease risk level increases. More specifically, for example, the threshold value corresponding to "LV0" is set to be "40%". The threshold value corresponding to "LV1" is set to be "35%". The threshold value corresponding to "LV2" is set to be "30%". The threshold value corresponding to "LV3" is set to be "25%". The threshold value corresponding to "LV4" is set to be "20%". The threshold value corresponding to "LV5" is set to be "15%". The threshold value corresponding to "LV6" is set to be "10%". The threshold value corresponding to "LV7" is set to be "5%".

If the threshold value is determined, it is judged whether or not at least one of the amount of change in the pulse rate HR and the amount of change in the blood flow volume BF exceeds the threshold value (step S53). Here, if at least one of the amount of change in the pulse rate HR and the amount of change in the blood flow volume BF does not exceed the threshold value (in other words, if both of the amount of change in the pulse rate HR and the amount of change in the blood flow volume BF do not exceed the threshold value) (the step S53: NO), it is judged that it is not in the high risk level state (step S54). In other words, it is judged that the risk of the decrease in blood pressure is not rapidly increasing.

On the other hand, if at least one of the amount of change in the pulse rate HR and the amount of change in the blood flow volume BF exceeds the threshold value (the step S53: YES), it is judged whether or not both of the amount of change in the pulse rate HR and the amount of change in the blood flow volume BF exceed the threshold value (step S55). Here, if both of the amount of change in the pulse rate HR and the amount of change in the blood flow volume BF do not exceed the threshold value (in other words, only at least one of the amount of change in the pulse rate HR and the amount of change in the blood flow volume BF exceeds the threshold value) (the step S54: NO), it is judged that it is in a high risk level A state (step S56). On the other hand, if both of the amount of change in the pulse rate HR and the amount of change in the blood flow volume BF exceed the threshold value (the step S55: YES), it is judged that it is in a high risk level B state (step S57).

Here, in particular, the high risk level B state is set to have a higher risk of occurrence of the decrease in blood pressure than the high risk level A state. Since it can be judged whether it is in the high risk level A state or in the high risk level B state, it is possible to accurately judge whether the risk is relatively low or high even in the case of the high risk level state.

Back in FIG. 3, if the high risk level state is judged, a result of the judgment of the high risk level is outputted from the high risk level judging device 160 (step S180). In accordance with the result of the judgment of the high risk level or the blood pressure decrease risk level determined by the risk level determining device 150, the controlling device 180 controls the operation of the dialysis apparatus 600 (step S190).

The controlling device 180 controls the rate of removal of water, ON/OFF, a dose of physiological saline solution and the like in the dialysis apparatus 600 such that the blood pressure of the living body 900 who is the patient hardly decreases. It is thus possible to effectively reduce the decrease in blood pressure of the living body 900 who is the patient.

Incidentally, the controlling device 180 not only controls the dialysis apparatus 600 but also may control the operation of the blood pressure decrease prediction apparatus 100 in accordance with various information obtained from the dialysis apparatus 600. For example, reflecting the information obtained from the dialysis apparatus 600, it is also possible to output the risk level and the high risk level state. More specifically, the controlling device 180 obtains signals for calculating the pulse rate and the blood flow volume, which are the parameters for determining the risk level; however, if the dialysis is not actually performed, the controlling device 180 can perform such control that the risk level and the high risk level state are not outputted, after receiving such information that the dialysis is not performed from the dialysis apparatus 600. This makes the operation of the apparatus more efficient.

Moreover, the blood pressure decrease prediction apparatus 100 may send signals to external equipment such as a sphygmomanometer and automatically operate the external equipment such as the sphygmomanometer, in accordance with the blood pressure decrease risk level determined by the risk level determining device 150.

Incidentally, the blood pressure decrease prediction apparatus 100 may be configured to display a graph which simultaneously illustrates the variations in the pulse rate calculated by the pulse rate calculating device 110, the blood flow volume calculated by the blood flow volume calculating device 120 and the pulse wave amplitude calculated by the pulse wave amplitude calculating device 130. In this case, the living body 900 who is the patient and the involved person who is the doctor or the nurse can intuitively recognize the risk of occurrence of the decrease in blood pressure, and it is practically very usable.

Moreover, the blood pressure decrease prediction apparatus 100 may not necessarily use the risk level determination table.

Moreover, the risk level determining device 150 may be configured to calculate a correlation value between the blood flow volume and the pulse wave amplitude and to determine the blood pressure decrease risk level on the basis of the calculated correlation value. For example, the risk level determining device 150 may determine the blood pressure decrease risk level to be a relatively high level if the blood flow volume and the pulse wave amplitude decrease in correlation with each other (i.e. the correlation value between the blood flow volume and the pulse wave amplitude is greater than a predetermined value, and the blood flow volume and the pulse wave amplitude decrease). In this case, it is possible to determine the blood pressure decrease risk level more appropriately.

Moreover, the risk level determining device 150 may be configured to determine the blood pressure decrease risk level on the basis of respective average deviations of the pulse rate, the blood flow volume and the pulse wave amplitude. In this case, it is possible to determine the blood pressure decrease risk level, more appropriately.

Moreover, the blood pressure decrease prediction apparatus 100 may be configured to determine the blood pressure decrease risk level on the basis of other information related to the decrease in blood pressure which is obtained from the blood flow waveform in addition to the pulse rate, the blood flow volume and the pulse wave amplitude. In this case, it is possible to determine the blood pressure decrease risk level more appropriately. In this case, the blood pressure decrease prediction apparatus 100 may determine the blood pressure decrease risk level on the basis of information related to the decrease in blood pressure of the patient who received the artificial dialysis in the past.

Moreover, the blood pressure decrease prediction apparatus 100 may be configured to prompt the living body 900 who is the patient and the involved person who is the doctor or the nurse to measure the blood pressure of the living body 900 by using the blood pressure meter, according to the blood pressure decrease risk level determined by the risk level determining device 150. Alternatively, the blood pressure decrease prediction apparatus 100 may be configured to measure the blood pressure of the living body 900 by using the blood pressure meter, according to the blood pressure decrease risk level determined by the risk level determining device 150.

As explained above, it is possible to predict the decrease in blood pressure of the living body 900, at an early stage, with causing little burden on the living body 900 and to appropriately determine the blood pressure decrease risk level which indicates the risk of occurrence of the blood pressure. Furthermore, it is possible to inform the living body 900 who is the patient and the involved person who is the doctor or the nurse, of the risk of the decrease in blood pressure. As a result, it is possible to treat the living body 900 who has the risk of the decrease in blood pressure, at an early stage.

The present invention is not limited to the aforementioned embodiments, but various changes may be made, if desired, without departing from the essence of the invention which can be read from the claims and the entire specification. A blood pressure decrease prediction apparatus, which involves such changes, is also intended to be within the technical scope of the present invention. The present invention can be also applied to an apparatus which predicts a symptom of dehydration of the living body. Namely, it is possible to determine a dehydration symptom level on the basis of the blood pressure decrease risk level.

### Description of Reference Signs

- 100: blood pressure decrease prediction apparatus
- 110: pulse rate calculating device
- 120: blood flow volume calculating device
- 130: pulse wave amplitude calculating device
- 140: blood pressure decrease predicting device
- 149: variation pattern table
- 150: risk level determining device
- 160: high risk level judging device
- 170: outputting device
- 180: controlling device
- 500: monitor
- 600: dialysis apparatus
- 800: blood flow waveform output apparatus
- 900: living body

## Claims

1. A blood pressure decrease prediction apparatus which is configured to predict a decrease in blood pressure of a living body,
the blood pressure decrease prediction apparatus comprising:
a pulse rate calculating device which is configured to calculate a pulse rate of the living body;
a blood flow volume calculating device which is configured to calculate a blood flow volume of the living body;
a risk level determining device which is configured to determine a risk level which indicates a risk of occurrence of the decrease in blood pressure, on the basis of a variation in at least one of the calculated pulse rate and the calculated blood flow volume during a first predetermined period; and
a high risk level judging device which is configured to judge that it is in a high risk level state in which the risk of occurrence of the decrease in blood pressure is higher than the determined risk level, if a variation range of at least one of the calculated pulse rate and the calculated blood flow volume during a second predetermined period which is shorter than the first predetermined period exceeds a threshold value which is determined in accordance with the determined risk level, wherein
the high risk level judging device judges that it is in the high risk level state in which the risk of occurrence of the decrease in blood pressure is higher, in a case where the variation ranges of both of the calculated pulse rate and the calculated blood flow volume exceed the threshold value which is determined in accordance with the determined risk level, in comparison with a case where the variation range of at least one of the calculated pulse rate and the calculated blood flow volume exceeds the threshold value which is determined in accordance with the determined risk level.

2. The blood pressure decrease prediction apparatus according to claim 1, wherein
the risk level determining device determines the risk level by referring to a predetermined risk level determination table in which the variation in at least one of the pulse rate and the blood flow volume is associated with the risk level.

3. The blood pressure decrease prediction apparatus according to claim 1, wherein
the pulse rate calculating device calculates the pulse rate of the living body on the basis of a blood flow waveform which represents a temporal variation in the blood flow volume of the living body, and
the blood flow volume calculating device calculates the blood flow volume of the living body on the basis of the blood flow waveform.

4. The blood pressure decrease prediction apparatus according to claim 1, further comprising a pulse wave amplitude calculating device which is configured to calculate pulse wave amplitude of the living body,
the risk level determining device determining the risk level which indicates the risk of occurrence of the decrease in blood pressure, on the basis of a variation in the calculated pulse wave amplitude, in addition to the variation in at least one of the calculated pulse rate and the calculated blood flow volume.

5. The blood pressure decrease prediction apparatus according to claim 4, wherein
the risk level determining device determines the risk level by referring to a predetermined risk level determination table in which a combination of the variation in at least one of the pulse rate and the blood flow volume and the variation in the pulse wave amplitude is associated with the risk level.

6. The blood pressure decrease prediction apparatus according to claim 4, wherein
the pulse rate calculating device calculates the pulse rate of the living body on the basis of a blood flow waveform which represents a temporal variation in the blood flow volume of the living body,
the blood flow volume calculating device calculates the blood flow volume of the living body on the basis of the blood flow waveform, and
the pulse wave amplitude calculating device calculates the pulse wave amplitude of the living body on the basis of the blood flow waveform.

7. The blood pressure decrease prediction apparatus according to claim 1, further comprising a first outputting device which is configured to output to the exterior the risk level determined by the risk level determining device.

8. The blood pressure decrease prediction apparatus according to claim 1, further comprising a second outputting device which is configured to output to the exterior the high risk level state determined by the high risk level judging device.

## Patentansprüche

1. Eine Blutdruckabfall-Vorhersagevorrichtung, die konfiguriert ist, um ein Abfallen des Blutdrucks eines lebenden Körpers vorherzusagen,
die Blutdruckabfall-Vorhersagevorrichtung aufweisend:
eine Pulsrate-Berechnungsvorrichtung, die konfiguriert ist, um eine Pulsrate des lebenden Körpers zu berechnen,
eine Blutflussvolumen-Berechnungsvorrichtung, die konfiguriert ist, um ein Blutflussvolumen des lebenden Körpers zu berechnen,
eine Risikolevel-Ermittlungsvorrichtung, die konfiguriert ist, um ein Risikolevel zu ermitteln, welches ein Risiko für das Auftreten eines Blutdruckabfalls anzeigt, auf der Basis einer Abweichung in mindestens einem von der berechneten Pulsrate und dem berechneten Blutflussvolumen, während einer ersten vorbestimmten Periode, und
eine hohes-Risikolevel-Beurteilungsvorrichtung, die ausgebildet ist, um zu beurteilen, dass ein hohes-Risikolevel-Zustand gegeben ist, in dem das Risiko des Auftretens des Blutdruckabfalls höher ist als das ermittelte Risikolevel, wenn ein Abweichungsbereich von mindestens einem von der berechneten Pulsrate und dem berechneten Blutflussvolumen während einer zweiten vorbestimmten Periode, die kürzer ist als die erste vorbestimmte Periode, einen Schwellenwert überschreitet, der in Übereinstimmung mit dem ermittelten Risikolevel ermittelt wird, und
wobei die hohes-Risikolevel-Beurteilungsvorrichtung beurteilt, dass der hohes-Risikolevel-Zustand gegeben ist, in dem das Risiko des Auftretens des Blutdruckabfalls höher ist, in einem Fall, bei dem die Abweichungsbereiche von sowohl der berechneten Pulsrate als auch des berechneten Blutflussvolumens den Schwellenwert überschreitet, der in Übereinstimmung mit dem ermittelten Risikolevel ermittelt wird, im Vergleich mit einem Fall, bei dem der Abweichungsbereich von mindestens einem von der berechneten Pulsrate und dem berechneten Blutflussvolumen den Schwellenwert überschreitet, der in Übereinstimmung mit dem ermittelten Risikolevel ermittelt wird.

2. Die Blutdruckabfall-Vorhersagevorrichtung gemäß Anspruch 1, wobei
die Risikolevel-Ermittlungsvorrichtung das Risikolevel durch Bezug auf eine vorbestimmte Risikolevel-Ermittlungstabelle, in der die Abweichung von mindestens einem von der Pulsrate und dem Blutflussvolumen dem Risikolevel zugeordnet ist, ermittelt.

3. Die Blutdruckabfall-Vorhersagevorrichtung gemäß Anspruch 1, wobei
die Pulsrate-Berechnungsvorrichtung die Pulsrate des lebenden Körpers auf der Basis eines Blutfluss-Kurvenverlaufs berechnet, der eine zeitliche Abweichung in dem Blutflussvolumen des lebenden Körpers repräsentiert, und
die Blutflussvolumen-Berechnungsvorrichtung das Blutflussvolumen des lebenden Körpers auf der Basis des Blutfluss-Kurvenverlaufs berechnet.

4. Die Blutdruckabfall-Vorhersagevorrichtung gemäß Anspruch 1, ferner aufweisend eine Pulskurvenamplitude-Berechnungsvorrichtung, die konfiguriert ist, um eine Pulskurvenamplitude des lebenden Körpers zu berechnen,
wobei die Risikolevel-Ermittlungsvorrichtung das Risikolevel, welches ein Risiko des Auftretens des Blutdruckabfalls anzeigt, auf der Basis einer Abweichung in der berechneten Pulskurvenamplitude, zusätzlich zu der Abweichung in mindestens einem von der berechneten Pulsrate und dem berechneten Blutflussvolumen, ermittelt.

5. Die Blutdruckabfall-Vorhersagevorrichtung gemäß Anspruch 4, wobei
die Risikolevel-Ermittlungsvorrichtung das Risikolevel bestimmt durch Bezug auf eine vorbestimmte Risikolevel-Ermittlungstabelle, in der eine Kombination aus der Abweichung in mindestens einem von der Pulsrate und dem Blutflussvolumen und der Abweichung in der Pulskurvenamplitude dem Risikolevel zugeordnet ist, ermittelt.

6. Die Blutdruckabfall-Vorhersagevorrichtung gemäß Anspruch 4, wobei
die Pulsrate-Berechnungsvorrichtung die Pulsrate des lebenden Körpers auf der Basis eines Blutfluss-Kurvenverlaufs, der eine zeitliche Abweichung in dem Blutflussvolumen des lebenden Körpers repräsentiert, berechnet,
die Blutflussvolumen-Berechnungsvorrichtung das Blutflussvolumen des lebenden Körpers auf der Basis des Blutfluss-Kurvenverlaufs berechnet, und
die Pulskurvenamplitude-Berechnungsvorrichtung die Pulskurvenamplitude des lebenden Körpers auf der Basis des Blutfluss-Kurvenverlaufs berechnet.

7. Die Blutdruckabfall-Vorhersagevorrichtung gemäß Anspruch 1, ferner aufweisend eine erste Ausgabevorrichtung, die konfiguriert ist, um das durch die Risikolevel-Ermittlungsvorrichtung ermittelte Risikolevel nach außen auszugeben.

8. Die Blutdruckabfall-Vorhersagevorrichtung gemäß Anspruch 1, ferner aufweisend eine zweite Ausgabevorrichtung, die konfiguriert ist, um den durch die hohes-Risikolevel-Beurteilungsvorrichtung ermittelten hohes-Risikolevel-Zustand nach außen auszugeben.

## Revendications

1. Appareil de prédiction de baisse de pression artérielle qui est configuré pour prédire une baisse de la pression artérielle d'un corps vivant,
l'appareil de prédiction de baisse de pression artérielle comprenant :
un dispositif de calcul de fréquence du pouls qui est configuré pour calculer une fréquence du pouls du corps vivant ;
un dispositif de calcul de volume de flux sanguin qui est configuré pour calculer un volume de flux sanguin du corps vivant ;
un dispositif de détermination de niveau de risque qui est configuré pour déterminer un niveau de risque qui indique un risque d'occurrence de la baisse de pression artérielle, sur la base d'une variation d'au moins l'un de la fréquence du pouls calculée et du volume de flux sanguin calculé au cours d'une première période prédéterminée ; et
un dispositif d'évaluation de niveau de risque élevé qui est configuré pour juger qu'il se trouve dans un état de niveau de risque élevé dans lequel le risque d'occurrence de la baisse de la pression artérielle est plus élevé que le niveau de risque déterminé, si une plage de variation d'au moins l'un de la fréquence de pouls calculée et du volume de flux sanguin calculé au cours d'une seconde période prédéterminée qui est plus courte que la première période prédéterminée dépasse une valeur de seuil qui est déterminée selon le niveau de risque déterminé, dans lequel
le dispositif d'évaluation de niveau de risque élevé juge qu'il se trouve dans l'état de niveau de risque élevé dans lequel le risque d'occurrence de la baisse de la pression artérielle est élevé, dans un cas où les plages de variation d'à la fois la fréquence de pouls calculée et le volume de flux sanguin calculé dépassent la valeur de seuil qui est déterminée selon le niveau de risque déterminé, par rapport à un cas où la plage de variation d'au moins l'un de la fréquence de pouls calculée et du volume de flux sanguin calculé dépasse la valeur de seuil qui est déterminée selon le niveau de risque déterminé.

2. Appareil de prédiction de baisse de pression artérielle selon la revendication 1, dans lequel
le dispositif de détermination de niveau de risque détermine le niveau de risque en se référant à une table de détermination de niveau de risque prédéterminée dans laquelle la variation d'au moins l'un de la fréquence de pouls et du volume de flux sanguin est associée au niveau de risque.

3. Appareil de prédiction de baisse de pression artérielle selon la revendication 1, dans lequel
le dispositif de calcul de fréquence de pouls calcule la fréquence de pouls du corps vivant sur la base d'une forme d'onde de flux sanguin qui représente une variation temporelle du volume de flux sanguin du corps vivant, et
le dispositif de calcul de volume de flux sanguin calcule le volume de flux sanguin du corps vivant sur la base de la forme d'onde de flux sanguin.

4. Appareil de prédiction de baisse de pression artérielle selon la revendication 1, comprenant en outre un dispositif de calcul d'amplitude d'onde de pouls qui est configuré pour calculer l'amplitude d'onde de pouls du corps vivant,
le dispositif de détermination de niveau de risque déterminant le niveau de risque qui indique le risque d'occurrence de la baisse de la pression artérielle, sur la base d'une variation de l'amplitude d'onde de pouls calculée, en plus de la variation d'au moins l'un de la fréquence de pouls calculée et du volume de flux sanguin calculé.

5. Appareil de prédiction de baisse de pression artérielle selon la revendication 4, dans lequel
le dispositif de détermination de niveau de risque détermine le niveau de risque en se référant à une table de détermination de niveau de risque prédéterminée dans laquelle une combinaison de la variation d'au moins l'un de la fréquence de pouls et du volume de flux sanguin et de la variation de l'amplitude d'onde de pouls est associée au niveau de risque.

6. Appareil de prédiction de baisse de pression artérielle selon la revendication 4, dans lequel
le dispositif de calcul de fréquence de pouls calcule la fréquence de pouls du corps vivant sur la base d'une forme d'onde de flux sanguin qui représente une variation temporelle du volume de flux sanguin du corps vivant,
le dispositif de calcul de volume de flux sanguin calcule le volume de flux sanguin du corps vivant sur la base de la forme d'onde de flux sanguin, et
le dispositif de calcul d'amplitude d'onde de pouls calcule l'amplitude d'onde de pouls du corps vivant sur la base de la forme d'onde de flux sanguin.

7. Appareil de prédiction de baisse de pression artérielle selon la revendication 1, comprenant en outre un premier dispositif de sortie qui est configuré pour délivrer en sortie vers l'extérieur le niveau de risque déterminé par le dispositif de détermination de niveau de risque.

8. Appareil de prédiction de baisse de pression artérielle selon la revendication 1, comprenant en outre un second dispositif de sortie qui est configuré pour délivrer en sortie vers l'extérieur l'état de niveau de risque élevé déterminé par le dispositif d'évaluation de niveau de risque élevé.
